# EUROPÄISCHE PATENTSCHRIFT

(11) **EP 0 401 600 B1**
(45) Veröffentlichungstag und Bekanntmachung des Hinweises auf die Patenterteilung: **03.01.1996**
(21) Anmeldenummer: 90109799.8
(22) Anmeldetag: 23.05.1990
(51) Int. Cl.: B65H 63/06, G01B 21/12, G01N 33/36, D01H 13/32, B65H 63/00

(54) **Vorrichtung zur Überwachung und/oder Messung von Parametern eines laufenden, faden- oder drahtartigen Prüfguts und Verfahren zum Betrieb der Vorrichtung**
Device for monitoring and/or measuring parameters of running test materials in form of thread or wire and method for operating the device
Dispositif pour surveiller et/ou mesurer des paramètres d'un matériau en mouvement à contrôler en forme de fil textile ou métallique et méthode pour le fonctionnement du dispositif

(30) Priorität: 07.06.1989 CH 2139/89
(43) Veröffentlichungstag der Anmeldung: 12.12.1990
(73) Patentinhaber: ZELLWEGER LUWA AG, CH-8610 Uster (CH)
(72) Erfinder: Laubscher, Hanspeter, CH-8625 Gossau/ZH (CH)

(56) Entgegenhaltungen:
- EP-A- 0 244 788
- AU-B- 563 012
- BE-A- 771 277
- CH-A- 439 796
- DD-A- 215 516

## Beschreibung

Die Erfindung betrifft eine Vorrichtung zur Ueberwachung und/oder Messung von Parametern eines laufenden, faden- oder drahtförmigen Prüfguts, mit einem zum Durchlauf des Prüfguts vorgesehenen Messspalt, an dessen beiden Seitenwänden je eine Teil eines kapazitiven Messorgans bildende Messelektrode vorgesehen ist.

Derartige auf dem kapazitiven Messprinzip beruhende Vorrichtungen sind heute millionenfach verbreitet, und zwar einerseits als sogenannte elektronische Garnreiniger und andererseits als Gleichmässigkeitsprüfer. Die elektronischen Garnreiniger, von denen hier diejenigen des Typs USTER AUTOMATIC der Zellweger Uster AG erwähnt seien, dienen zur Erfassung störender Garnfehler, wie beispielsweise kurzen Dickstellen, Dünnstellen und häufigen Dick-Dünnstellen (Moiré). Gleichmässigkeitsprüfer wie der USTER TESTER der Zellweger Uster AG (USTER - eingetragenes Warenzeichen der Zellweger Uster AG) dienen zur Erfassung und Analyse der Schwankungen des Gewichtes pro Längeneinheit an Bändern, Vorgarnen und Garnen.

Das kapazitive Messprinzip hat wegen seiner hohen Messgenauigkeit und seiner über viele Jahre gleichbleibenden Empfindlichkeit eine sehr starke Verbreitung gefunden. Neben den auf diesem Prinzip basierenden Vorrichtungen werden auch noch optische Messköpfe verwendet, mit denen der Durchmesser des Prüfgut bestimmt wird. Diese optischen Messköpfe werden insbesondere dann verwendet, wenn kapazitive Messköpfe nicht eingesetzt werden können. Dies ist beispielsweise bei der Untersuchung von elektrisch leitfähigen Garnen der Fall.

Unabhängig von der Art des verwendeten Messprinzips weisen die Messköpfe eine gewisse, prinzipiell und konstruktiv bedingte, Abhängigkeit von Fremdeinflüssen, wie beispielsweise Feuchtigkeit, Form des Garnquerschnitts, Positionsabhängigkeit, Materialeinfluss, und so weiter, auf, die mit der gegebenen Technologie nicht eliminiert oder verkleinert werden kann, obwohl ein starkes Interesse daran bestünde. Ein weiteres bisher noch ungelöstes Problem ist ein für alle Arten von Garnen verwendbarer Universalmesskopf mit allen Vorteilen des kapazitiven Messprinzips. Schliesslich wäre es mit fortschreitender Automatisierung auch wünschenswert, wenn eine Art von sich selbst kontrollierendem Messkopf zur Verfügung stünde.

Aus der AU-B-563012, die dem Oberbegriff des Anspruchs 1 entspricht, ist zwar eine Vorrichtung zur Ermittlung des Drehwinkels eines Garnes bekannt, mit der der Garndurchmesser in einem optischen Sensor und die Garnmasse in einem garnaufwärts angeordneten kapazitiven Sensor erfasst werden. Der optische und der kapazitive Sensor sind aber Teile eigenständiger, räumlich getrennter Geräte, die verschiedenartige Grössen messen und ihre Signale getrennt voneinander abgeben, so dass die Abhängigkeit von Fremdeinflüssen für jeden Sensor bestehen bleibt.

Durch die Erfindung sollen alle diese Aufgaben gelöst werden. Es soll also ein Messkopf angegeben werden, dessen Abhängigkeit von Fremdeinflüssen kleiner ist als bei den bekannten Messköpfen, der universell verwendbar ist, und der sich selbst kontrolliert.

Diese Aufgabe wird erfindungsgemäss durch die Merkmale des Anspruchs 1 gelöst.

Praktische Versuche haben gezeigt, dass beide Messorgane in einer Weise zusammenwirken, dass die Abhängigkeit von Fremdeinflüssen und dadurch bedingte Genauigkeitsschwankungen gegenüber den bekannten Messköpfen verkleinert werden. Die universelle Verwendbarkeit der erfindungsgemässen Messköpfe ist evident und die angestrebte Selbstkontrolle ist dadurch gegeben, dass Abweichungen zwischen den von den beiden Messorganen gelieferten Ergebnissen dahingehend interpretiert werden, dass eines der beiden Messorgane nicht fehlerfrei arbeitet.

Ueber die genannten Eigenschaften hinaus hat der erfindungsgemässe Messkopf noch den zusätzlichen Vorteil, dass er die On-line Messung und Ueberwachung von Parametern gestattet, welche bisher nicht, oder zumindest nicht auf derart einfache Weise, überwacht werden konnten. So können neben den üblicherweise überwachten Parametern Querschnitt und Durchmesser zusätzlich beispielsweise auch noch Bauschigkeit, Haarigkeit und Feuchtigkeit überwacht und gemessen werden. Eine weitere interessante und bisher nicht mögliche Applikation ergibt sich aus der kombinierten Auswertung der Werte der beiden Messorgane: Das kapazitive Messorgan misst bekanntlich den Querschnittsverlauf, oder genauer gesagt, das Gewicht des Prüfguts pro Längeneinheit, und das optische Messorgan misst den Durchmesser. Die Kombination beider Arten von Messwerten liefert nun eine Aussage über das Gewicht pro Volumen, also eine mit der physikalischen Dichte vergleichbare Grösse. Aus dieser kann wieder auf andere Grössen, wie beispielsweise auf die Garndrehung geschlossen werden.

Aus dieser beispielhaften und keineswegs abschliessenden Aufzählung ist ersichtlich, dass der erfindungsgemässe Messkopf eine Reihe von überraschenden Eigenschaften aufweist und insbesondere die Messung und Ueberwachung von Messgrössen ermöglicht, die bisher mit den bekannten einfachen Messköpfen, seien diese nun kapazitive oder optische, nicht gemessen werden konnten.

Die Erfindung betrifft weiter ein Verfahren zum Betrieb der genannten Vorrichtung. Dieses Verfahren ist dadurch gekennzeichnet, dass ein automatischer Abgleich der beiden Messorgane auf gleiche Empfindlichkeit erfolgt.

Im folgenden wird die Erfindung anhand von Ausführungsbeispielen und der Zeichnungen näher erläutert; es zeigen:
- Fig. 1: eine schematische Schnittdarstellung eines ersten Ausführungsbeispiels einer erfindungsgemässen Vorrichtung mit der Schnittebene quer zur Fadenlaufrichtung,
- Fig. 2: eine Detailvariante des zweiten Ausführungsbeispiels von Fig. 1.

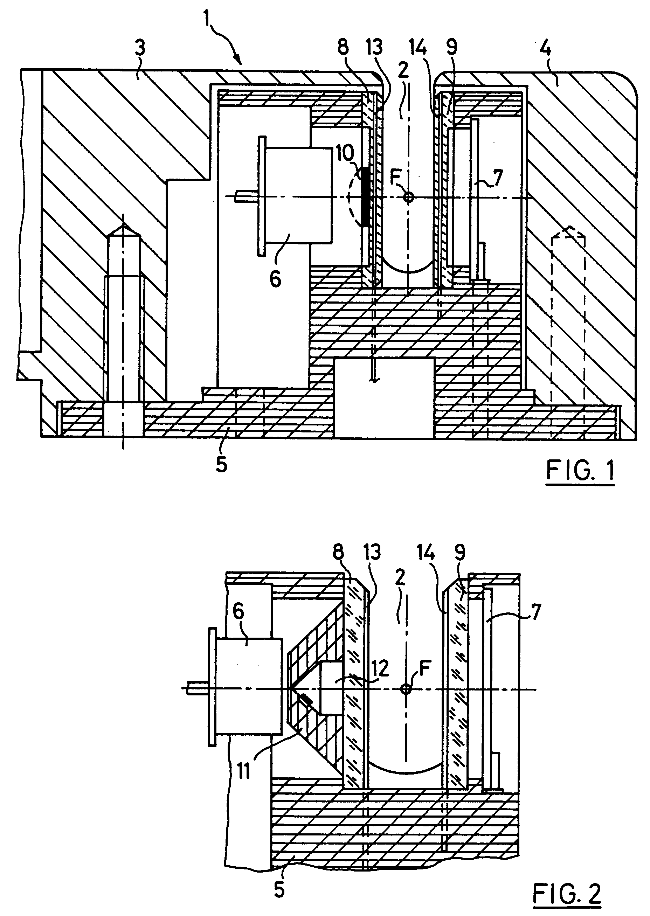

Die in den Figuren dargestellten Vorrichtungen dienen zur Ueberwachung und/oder Messung von Parametern von laufenden Fäden, insbesondere zur sowohl optischen als auch kapazitiven Erfassung von deren Durchmesser und Querschnitt. Diese auch als Messköpfe bezeichneten Vorrichtungen weisen je ein Gehäuse 1 mit einem Messspalt 2 auf, durch welchen der zur vermessende Faden F läuft. Faden bezeichnet in diesem Zusammenhang ein langgestrecktes Prüfgut von der Art eines Fadens oder Garns oder eines textilen Bandes bis hin zu einem Draht. Die Darstellung erfolgt jeweils in einem vergrösserten Massstab von etwa 5:1; bei dem in Fig. 2 dargestellten Ausführungsbeispiel ist der Messspalt 2 breiter als bei dem Ausführungsbeispiel der Fig. 1.

Das einstückig aus Kunststoff gespritzte Gehäuse 1 ist in Form einer Schachtel mit offenem Boden ausgebildet. Es wird durch eine Ausnahmung für den Messspalt 2 in zwei Hälften 3 und 4 unterteilt. In den offenen Gehäuseboden ist eine Trägerplatte 5 für die optischen und elektronischen Teile der Vorrichtung eingesetzt und mit dem Gehäuse 1 verschraubt.

Auf der Trägerplatte 5 ist im Bereich der in den Figuren linken Gehäusehälfte 3 eine Lichtquelle 6, vorzugsweise eine Leuchtdiode angeordnet, welche Licht auf eine im Bereich der rechten Gehäusehälfte 4 angeordnete Fotodiode 7 aussendet. Der Messspalt 2 ist gegen die Leuchtdiode 6 und gegen die Fotodiode 7 hin je durch eine Streuscheibe 8 bzw. 9 abgedeckt, wodurch im Messspalt 2 eine diffuse Beleuchtung entsteht, welche auch als diffuses Licht auf die Fotodiode 7 fällt. Die Streuscheibe 9 vor der Fotodiode 7 kann auch als Filterscheibe zur Abschirmung des Umgebungslichts ausgebildet sein oder sie kann gleichzeitig als Streu- und als Filterscheibe dienen.

Wenn ein den Messspalt 2 durchlaufender Faden F eine von einem Garnfehler, beispielsweise von einer Dick- oder einer Dünnstelle herrührende Querschnittsänderung aufweist, dann ändert sich die Abschattung der Fotodiode 7 und dementsprechend deren Ausgangssignal. Anhand dieser Aenderung des Ausgangssignals der Fotodiode 7 kann nun entweder die Fehlstelle als solche bloss registriert, oder es kann der laufende Faden F angehalten und die Fehlstelle beseitigt werden.

Zur Erzielung eines homogenen Beleuchtungsfeldes im Messspalt 2 ist zwischen der Leuchtdiode 6 und der Streuscheibe 8 eine Blende 10 (Fig. 1) oder ein kegelstumpfförmiger Lichtleiter 11 mit einer Vertiefung 12 (Fig. 2) vorgesehen. Diese Elemente werden hier nicht weiter erläutert; es wird in diesem Zusammenhang auf die EP-A-244 788 verwiesen, wo der optische Teil des in den Figuren dargestellten Messkopfs ausführlich beschrieben ist.

Wie den Figuren zu entnehmen ist, ist zusätzlich zum bisher beschriebenen optischen Messorgan noch ein sogenanntes kapazitives Messorgan vorgesehen, wobei die einzelnen Figuren verschiedene Möglichkeiten der Anordnung des kapazitiven Messorgans relativ zum optischen zeigen. Messköpfe mit kapazitiven Messorganen sind in den elektronischen Garnreinigern USTER AUTOMATIC und in den Gleichmässigkeitsprüfern USTER TESTER der Zellweger Uster AG seit Jahrzehnten weltweit verbreitet und werden daher als bekannt vorausgesetzt. Aus der Vielzahl von derartige Messköpfe betreffenden Patenten seien die US Patente 2 516 768 und 3 009 101 genannt.

In den Figuren ist das kapazitive Messorgan durch zwei Kondensatorplatten 13, 14 symbolisiert; auf eine detaillierte Darstellung wird wegen der Bekanntheit des kapazitiven Messprinzips verzichtet. Die Fig. 1 und 2 zeigen je eine Anordnung, bei welcher die Messzonen des optischen und des kapazitiven Messorgans zusammenfallen.

Gemäss Fig. 1 sind die Kondensatorplatten 13 und 14 durch je eine in die Streuscheiben 8 beziehungsweise 9 eingebettete, elektrisch leitfähige und für das optische Messorgan "durchsichtige" Schicht aus Metall oder Kunststoff gebildet. Dabei kann jede Streuscheibe sandwichartig ausgebildet und die genannte Schicht auf den einen Teil aufgedampft oder durch Zerstäuben (sogenanntes aufsputtern) aufgebracht sein. Eine andere Möglichkeit besteht darin, die die Kondensatorplatten 13, 14 bildenden Schichten durch ein feinmaschiges Netz aus einem geeigneten Material, wie beispielsweise Metall, zu bilden.

Beim Ausführungsbeispiel von Fig. 2 sind die die Kondensatorplatten 13, 14 bildenden Schichten nicht in die Streuscheiben 8, 9 eingebettet, sondern die Streuscheiben sind an einer Seite mit der Schicht überzogen. Diese Seite kann, so wie in der Figur dargestellt, die dem Faden F zugewandte, sie kann aber grundsätzlich auch die vom Faden F abgewandte Seite sein. Für die Ausbildung der Schichten und für deren Material gelten die Ausführungen zu Fig. 1.

Da beide Messsysteme, das kapazitive und das optische, in der Regel verschiedene Empfindlichkeit aufweisen werden, müssen sie auf gleiche Empfindlichkeit abgeglichen werden. Dies erfolgt vorzugsweise automatisch vor dem Start des Messung, und zwar mittels Korrelation oder ähnlichen Verfahren, mit oder ohne den statischen Signalanteil.

Zur Erzeugung eines genaueren Summensignals werden vorzugsweise die beiden Messsignale gemittelt, und zwar nach erfolgter Signalumformung und Filterung im Zeit- oder Frequenzbereich zur Unterdrückung der jeweils ungünstigeren Fremdeinflüsse. Beim Einlegen des Fadens wird der Nullpunkt der beiden Messorgane abgeglichen. Driften wird durch das stabilere Messorgan korrigiert und die Verstärkung wird in den Fällen, wo die Feuchtigkeit variieren kann, vom optischen Messorgan her abgeleitet. Unregelmässigkeiten des laufenden Fadens F, wie Dickstellen, Dünnstellen und dergleichen, werden durch Mittelung der Signale beider Messorgane bestimmt. Die Bestimmung von Nissen erfolgt aus den Signalen des optischen Messorgans, durch Ausfiltern im Zeit- oder Frequenzbereich.

Zur Feststellung der Aenderung von Fremdeinflüssen wird nicht die Summe, sondern die Differenz der beiden Messsignale nach selektiver Verstärkung der Fremdeinflüsse gebildet.

Die Verwendung beider Messorgane ermöglicht auch eine Selbstkontrolle des Messkopfs durch Vergleich von einzelnen ausgefilterten Signalkomponenten und deren Ueberprüfung auf Plausibilität. Zeigt eine einzelne Komponente oder eine Kombination solcher Komponenten eine aussergwöhnliche Abweichung, dann wird auf eine Fehlfunktion eines Messorgans geschlossen und auf dasjenige mit dem vermuteten korrekten Messwert umgeschaltet. Gegebenenfalls kann auch nur der gestörte Signalanteil umgeschaltet oder ein Alarm ausgelöst werden.

Die Signale der beiden Messorgane können so ausgewertet werden, dass Aussagen über weitere Messgrössen möglich sind, beispielsweise über Bauschigkeit, Haarigkeit, Feuchtigkeit, Fremdfasern, Drehung, spezifisches Gewicht, und dergleichen.

Schliesslich wird sich in aller Regel durch die gleichzeitige Verwendung beider Arten von Messorganen eine Erhöhung der Messgenauigkeit ergeben, indem sich die Genauigkeitsschwankungen beider Systeme gegenseitig zumindest teilweise eliminieren.

## Patentansprüche

1. Vorrichtung zur Ueberwachung und/oder Messung von Parametern eines laufenden, faden- oder drahtförmigen Prüfguts, mit einem zum Durchlauf des Prüfguts vorgesehenen Messspalt (2) an dessen beiden Seitenwänden je eine, Teil eines kapazitiven Messorgans bildende Messelektrode (13, 14) vorgesehen ist, dadurch gekennzeichnet, dass zusätzlich zum kapazitiven ein optisches Messorgan mit einer an der einen Seite des Messspalts angeordneten Lichtquelle (6) und mit einem an der anderen Seite des Messspalts angeordneten fotoelektrischen Element (7) so vorgesehen ist, dass die Messelektroden des kapazitiven Messorgans im Strahlengang des optischen Messorgans angeordnet sind, dass der Messspalt zu beiden Seiten durch ein optisches Element (8, 9) begrenzt ist, das die Messelektroden trägt und dass beide Messorgane Teil eines gemeinsamen Messkopfs bilden.

2. Vorrichtung nach Anspruch 1, dadurch gekennzeichnet, dass die beiden Messorgane auf einem gemeinsamen Träger (5) integriert und/oder in einem gemeinsamen Gehäuse (1) angeordnet sind.

3. Vorrichtung nach Anspruch 1, dadurch gekennzeichnet, dass die beiden Messorgane Messzonen aufweisen, die sich zumindest partiell, überdecken.

4. Vorrichtung nach Anspruch 1, dadurch gekennzeichnet, dass als optisches Element eine Streuscheibe (8, 9) vorgesehen ist.

5. Vorrichtung nach Anspruch 4, dadurch gekennzeichnet, dass jede Messelektrode (13, 14) gitter- oder siebartig ausgebildet und in die zugehörige Streuscheibe (8 bzw. 9) eingebettet oder auf diese appliziert ist.

6. Vorrichtung nach Anspruch 4, dadurch gekennzeichnet, dass jede Messelektrode (13, 14) durch eine für das optische Messorgan transparente folienartige Schicht gebildet ist.

7. Vorrichtung nach Anspruch 6, dadurch gekennzeichnet, dass die folienartige Schicht durch eine auf die Streuscheibe (8, 9) aufgedampfte oder aufgesprühte Metallschicht gebildet ist.

8. Vorrichtung nach Anspruch 6, dadurch gekennzeichnet, dass die folienartige Schicht durch eine auf die Streuscheibe (8, 9) applizierte Schicht aus elektrisch leitendem Kunststoff gebildet ist.

9. Verfahren zum Betrieb der Vorrichtung gemäss Anspruch 1, dadurch gekennzeichnet, dass ein automatischer Abgleich der beiden Messorgane auf gleiche Empfindlichkeit erfolgt.

10. Verfahren nach Anspruch 9, dadurch gekennzeichnet, dass der Abgleich durch Korrelation der Signale der beiden Messorgane erfolgt.

11. Verfahren nach Anspruch 10, dadurch gekennzeichnet, dass die Signale der beiden Messorgane getrennt umgeformt und gefiltert und anschliessend zur Erzeugung eines Summensignals gemittelt werden.

12. Verfahren nach einem der Ansprüche 9 bis 11, dadurch gekennzeichnet, dass von den Signalen jedes Messorgan einzelne Komponenten ausgefiltert und auf das Auftreten aussergewöhnlicher Abweichungen untersucht werden.

13. Verfahren nach Anspruch 12, dadurch gekennzeichnet, dass das Auftreten aussergewöhnlicher Abweichungen als Fehlfunktion eines Messorgans interpretiert wird und die Signale dieses Messorgans bei der Auswertung nicht berücksichtigt werden.

## Claims

1. Device for monitoring and/or measuring parameters of a running test material in thread or wire form, having a measuring gap (2), which is provided for the passage of the test material and on each of the two side walls of which is provided a measuring electrode (13, 14) forming part of a capacitive measuring element, characterized in that an optical measuring element having a light source (6) arranged on one side of the measuring gap and having a photoelectric element (7) arranged on the other side of the measuring gap is provided in addition to the capacitive measuring element in such a way that the measuring electrodes of the capacitive measuring element are arranged in the beam path of the optical measuring element, that the measuring gap is bounded on both sides by an optical element (8, 9) which supports the measuring electrodes and that both measuring elements form part of a common measuring head.

2. Device according to Claim 1, characterized in that the two measuring elements are integrated on a common carrier (5) and/or are arranged in a common housing (1).

3. Device according to Claim 1, characterized in that the two measuring elements have measuring zones which overlap at least partially.

4. Device according to Claim 1, characterized in that a diffusing screen (8, 9) is provided as the optical element.

5. Device according to Claim 4, characterized in that each measuring electrode (13, 14) is of lattice-like or sieve-like design and is embedded in the associated diffusing screen (8 or 9) or applied to the latter.

6. Device according to Claim 4, characterized in that each measuring electrode (13, 14) is formed by a film-like layer which is transparent for the optical measuring element.

7. Device according to Claim 6, characterized in that the film-like layer is formed by a metal layer vapour-deposited on or sprayed onto the diffusing screen (8, 9).

8. Device according to Claim 6, characterized in that the film-like layer is formed by a layer of electrically conductive plastic applied to the diffusing screen (8, 9).

9. Method for operating the device according to Claim 1, characterized in that automatic matching of the two measuring elements to the same sensitivity is effected.

10. Method according to Claim 9, characterized in that the matching is effected by correlation of the signals of the two measuring elements.

11. Method according to Claim 10, characterized in that the signals of the two measuring elements are separately reshaped and filtered and then averaged to produce a composite signal.

12. Method according to one of Claims 9 to 11, characterized in that individual components of the signals of each measuring element are filtered out and tested for the occurrence of unusual deviations.

13. Method according to Claim 12, characterized in that the occurrence of unusual deviations is interpreted as a malfunction of one measuring element and the signals of this measuring element are not considered in the evaluation.

## Revendications

1. Dispositif pour surveiller et/ou mesurer des paramètres d'un matériau à contrôler en mouvement, en forme d'un fil textile ou d'un fil métallique, avec une fente de mesure (2) prévue pour le passage du matériau à contrôler, aux deux côtés latéraux de laquelle est prévue chaque fois une électrode de mesure (13,14) formant une partie d'un organe de mesure capacitif, caractérisé en ce que, supplémentairement à l'organe de mesure capacitif, est prévu un organe de mesure optique comprenant une source de lumière (6) disposée sur un côté de la fente de mesure et un élément photoélectrique (7) disposé sur l'autre côté de la fente de mesure, que les électrodes de mesure de l'organe de mesure capacitif sont placées dans la trajectoire des rayons de l'organe de mesure optique, que la fente de mesure est limitée aux deux côtés par un élément (8,9) qui porte les électrodes de mesure et que les deux organes de mesure forment une partie d'une tête de mesure commune.

2. Dispositif selon la revendication 1, caractérisé en ce que les deux organes de mesure sont incorporés à un support commun (5) et/ou sont disposés dans un boîtier commun (1).

3. Dispositif selon la revendication 1, caractérisé en ce que les deux organes de mesure comportent des zones de mesure qui se recouvrent au moins partiellement.

4. Dispositif selon la revendication 1, caractérisé en ce qu'un disque diffuseur (8,9) est prévu comme élément optique.

5. Dispositif selon la revendication 4, caractérisé en ce que chaque électrode de mesure (13,14) est réalisée sous forme d'une grille ou d'un tamis et est noyée dans le disque diffuseur correspondant (8 ou 9) ou appliquée à celui-ci.

6. Dispositif selon la revendication 4, caractérisé en ce que chaque électrode de mesure (13,14) est formée par une couche en forme d'un film transparent pour l'organe de mesure optique.

7. Dispositif selon la revendication 6, caractérisé en ce que la couche en forme d'un film est formée sur le disque diffuseur (8,9) par métallisation sous vide ou par pulvérisation.

8. Dispositif selon la revendication 6, caractérisé en ce que la couche en forme de film est formée par une couche en matière plastique électriquement conductrice, appliquée au disque diffuseur (8,9).

9. Procédé pour faire fonctionner le dispositif selon la revendication 1, caractérisé en ce que l'on fait un réglage automatique des deux organes de mesure à la même sensibilité.

10. Procédé selon la revendication 9, caractérisé en ce que le réglage se fait par corrélation des signaux des deux organes de mesure.

11. Procédé selon la revendication 10, caractérisé en ce que les signaux des deux organes de mesure sont convertis séparément et filtrés et ensuite on établit la moyenne pour produire un signal cumulé.

12. Procédé selon l'une des revendications 9 à 11, caractérisé en ce que l'on sépare par filtrage des composantes des signaux de chaque organe de mesure et examine celles-ci sur la présence d'écarts extraordinaires.

13. Procédé selon la revendication 12, caractérisé en ce que la présence d'écarts extraordinaires est interprétée comme fonction erronée d'un organe de mesure et les signaux de cet organe de mesure ne sont pas pris en compte lors de l'exploitation.
